# EUROPEAN PATENT APPLICATION

(11) **EP 0 774 264 A1**
(43) Date of publication of application: **21.05.1997**
(21) Application number: 96308395.1
(22) Date of filing: 20.11.1996
(51) Int. Cl.: A61L 9/01

(54) **Air purifying agent and air purifying method**

(30) Priority: 20.11.1995 JP 325028/95; 20.11.1995 JP 309232/95; 20.11.1996 JP 309232/96
(71) Applicant: Espo, Ltd., Chiba-shi, Chiba (JP)
(72) Inventor: Kobayashi, Nobuo, Mihama-ku, Chiba-shi (JP); Hattori, Fujio, Mihama-ku, Chiba-shi (JP); Iida, Kikuo, Wakaba-ku, Chiba-shi, (JP); Adachi, Koichiro, Kashiwa-shi, Chiba (JP)
(74) Representative: Perry, Robert Edward

(57) **Abstract**

An air refreshing agent containing lithium chloride as an essential component and also at least one essential component selected from a group consisting of (A) water-soluble and/ or water-swelling natural and / or synthetic high molecule compound excluding a water-soluble high molecule compound having a molecular weight of 1.0 x 1⁷ or less when measured according to the intrinsic viscosity method and comprising acrylamide as a constituent monomer; (B) a mixture of any of zinc carbonate, zinc hydroxide, zinc gluconate, calcium lactate, calcium glycerinophosphate, calcium gluconate, ferrous nitrate, ferrous lactate, ferrous gluconate, copper gluconate, calcium chloride, a mixture of water-insoluble calcium salt and a gluconodelta lactone, and further at least 2-amino-2-methyl-1-propanol salt and/ or alkali metallic salt of acids such as phosphorus hydroxide, citric acid, lactic acid, DL-malic acid, fumaric acid, succinic acid, gluconic acid; (C) carbonate and/ or bicarbonate of alkali metal; (D) acids such as citric acid, lactic acid. DL-malic acid, fumaric acid, succinic acid, gluconic acid, and phosphorus hydroxide; (E) activated carbon, and (F) anticeptics, germicide, antibiotics, mildew proofing agent, tickcide, pesticide, agriculture chemicals, flame reterdant, and sulfuric sodium sulfats anhydride, said air refreshing agent in any of forms of an aqueous solution, gel, powder, or deposited on textile, woods, activated carbon or the like, and also an air refreshing method using the same.

## Description

### FIELD OF THE INVENTION

The air refreshing agent and air refreshing method according to the present invention related to the two technological fields.
(A) Removal of air contaminants: Firstly, the present invention is utilized to remove malodorous and toxic gas molecules each having a diameter in a range from around 0.3 nm to 0.8 nm, smoke generated in a fire, or the like and having a particle diameter in a range from around 0.001 µm to 100 µm, hazardous aerosols or suspended particulate matters, microorganisms or spores thereof or the like ( which sometimes are genetically described as air-contaminants hereinafter) in confined or semi-confined rooms or spaces within a building where normal human living or works are performed and also where the accumulation of the air contaminants is not desirable. The rooms or spaces as described above include a living room, a working room, a lavatory, a dining room, a cocking room, a food shop or a storage chamber thereof, a game shop, a sickroom, an surgery room or a cockpit, a food processing room, a guest room of a hotel; a passenger room, a control room, or a working room in a vehicle such as a train, an airline or a ship; an animal breeding room, a refrigerator, a cupboard for shoes, a beauty treatment room, a dressing room in a sport-related facility (which are sometimes described as air-contaminated spaces hereinafter).
(B) Prevention of dispersion of useful, but poisonous substances: Secondly, the present invention intends to reduce or retard diffusion of hazardous substances into the air in a case where antiseptics, germicide, antibiotics, mildew proofing agent, tickcide(or mitecide), pesticide, agricultural chemicals, flame retardent, and other useful but hazardous substances within a building, over plants or in soil, and further to improve the utilization rate and prolong the term of effect by retarded drying of mist of microgel adhered to these object materials, and further to prevent these hazardous materials from being deposited onto a human body and/or inhaled by a user.

### RELATED BACKGROUND TECHNOLOGY

(a) A bug filter is used for collecting dust having a particle diameter of several µm or more among the air contaminants described above, but the bug filter is ineffective against smaller air contaminants than these.
(b) A chemical cleaning method with a scrubber or a splay device has widely been employed against sulfuric acid mist or hazardous aerosol each having a particle diameter in a range from around 1 µm to 10 µm, but size of the devices is large, and requires large energy furthermore the maintenance work for the devices is not easy.
(c) For smoke having a particle diameter in a range around 0.01 µm to 5 µm or a soot or incompletely burnt hydrocarbon discharged from engines in an underground parking place, an electro-static dust collector or an exhausting method diluting with a large quantity of fresh air has widely been employed, but the device or the method is ineffective for capturing hazardous gasses or mal odor such as aerosol, SOx, NOx having a particle diameter of around 10 µm or less.
(d) For removing or preventing diffusion of harmful viruses having a particle diameter in a range from around 0.003 µm to around 0.05µm or harmful microorganisms having a particle diameter in a range from around 0.03 µm to around 30 µm, spraying or coating of germcide, mildew resistant agent, or a microorganism proliferation inhibitor (which may genetically be described as germcide hereinafter) and/ or diffusion of ozone gas, chloric acid gas, formaldehyde, all of which are harmful, or irradiation of ultraviolet ray have widely been used. With any of the methods as described above, harmful or mal odorous gasses are generated against the user's intention.
(e) Against air contaminants from molecules up to aerosol having a particle diameter in a wide range, a method of refreshing air by contacting the air to or passing the air through activated carbon subjected to processing with various chemicals has widely been employed, but the effect under a low humidity becomes lower in some types of chemicals or the use efficiency becomes lower when covered by air contaminants having a large particle diameter.
(f) When a solution, an emulsion or powder preparation of useful but poisonous substance is sprayed over an object material, fine particles in the solution quickly dries or the powder preparation is diffused by wind, so that a percentage of particles not adhered to the object material and diffused in the air is high, so that the working environment is contaminated or workers, resident, and cattle inhale agricultural chemicals or the like, and further after the components deposited on the object material are dried, the components may disadvantageously be blown off by wind.
(g) An aqueous cast and gelled air refreshing agent dries quickly, and even if the components remain sufficiently, the efficiency is lost due to shortage of water content in many cases.
(h) If a powdery or other solid type of air-refreshing agent is used in a space under low humidity, sometimes any chemical reaction does not occur, and the effect is not shown at all.

As described above, in these conventional technologies, in spite that a respiratory organ of a human or an animal inhales these various types of air contaminants simultaneously, each substance is separately treated according to a particle diameter thereof or the chemical or physical property for removing the substance.

### DISCLOSURE OF THE INVENTION

An object of the present invention is to provide a an air refreshing agent (which is described as a chemical hereinafter) and an air refreshing method as described below which have not been attained with the conventional technology. Firstly, the main problems to be solved in the present invention in relation to the chemical are as follows.
(a) To provide a chemical which can refresh air contaminants having a particle diameter in a wide range by moistening the chemical to a desired degree and by means of contact or absorption.
(b) To reduce poisonous and hazardous air contaminants by spraying a chemical containing a specific high molecular compound especially in a case of a fire to suppress smoke filled in a room, to detoxify harmful gasses, to retard flashover, or to suppress evaporation of water.
(c) To enhance a chemical reactivity of contained components, and to preserve effectiveness of a physical or a physicochemical air refreshing effect or efficacy of agricultural chemicals to harmful microorganisms as long as possible even under high temperature or low humidity by raising a contact ratio between air contaminants and the chemical, prolonging an available period of the chemical, and preventing evaporation of the contained components.
(d) To impart a directionality to a sprayed mist of aqueous and toxic chemical such as agricultural chemical containing a high molecular compound previously added thereto when injected from a nozzle by preventing the scatter of the mist into the air due to the enlarged particle size therein and by an increased rate of adhesion onto an object material inspraying, and also to impart a sustained releaseing effect reducing the concentrationas well as the amount to be sprayed or to be sprinkled by the micro-capsulizing effect of the high molecular compound, furthrtmore to reduce the leach out of the agricultural chemicals and the like by rain water, resuspension of them by wind, envioromental contamination, and toxicity level of the chemicals to workers, residents and domestic animals when sprayed and even after the mist is dried.
(e) To allow addition of lithium chloride which is very salty but has low toxicity to a cast and gelled type deodorizer or germcide for the purpose to prevent persons having low discernment or pet animals from eating the deodorizer or germcide as well as to prevent or retard proliferation of microorganisms.
(f) To enable provision of chemicals each particularly effective to a specific air contaminant by the use of a physically and/or chemically coagulating or absorbing force of a high molecular compound, or by blending various types of additives therein to impart the effect for preventing drying, high reactivity or buffering effect,
(g) To enable utilization of lithium chloride and an amphoteric high molecular compound having a molecular weight above 1.0 x 10⁷ such as polyacrylamide or a preparation with germcide, agricultural chemical blended therein for realization of a strong germicidal , pesticidal, and air-refreshing effects even under a low density as well as for continuous air refreshment in a sickroom , an animal breeding room or the like.
(h) To reduce the unpleasantness or bad influence to residents or human health by volatile organic compounds such as formaldehyde generated from plywoods or interior paneling used in new furniture or new residence as well as of mal odorous materials contained in plasticizers, coating , moth proofing agents, and other chemicals by continuous removing them through coating, treating the material with these chemicals by impregnating, drying and/ or coating according to the present invention
(i) To improve selective reactivity of activated carbon by adding an appropriate quantity of lithium chloride for maintaining moisture therein to a hazardous gas with a chemical
(j) To improve defects in the conventional air conditioning system which may give bad effects to a living or working spaces and to health of residents' neighbors' or petanimals because the conventional air conditioning apparatus is large and has a complicated mechanism, consumes a large electric power, generates large noises, and also because dewdrops are easily formed by excessive cooling or excessive damp-proofing, molds easily proliferate, and a lot of microorganisms and spores thereof are discharged from the air exhaust pipe.
(k) To reduce a load to air-conditioning facility for an entire building and also to enable size reduction as well as energy consumption by locally absorbing or making lower a concentration of air contaminants in a contaminated space within the building and especially at a site with high concentration of air contaminant by using a chemicalaccording to the present invention having high drying resistance.

To achieve the objects as described above, the present invention provides a chemical characterized in that:
(1) the molecular weight measured by the intrinsic viscosity method is in a range from 3.0 x 10⁶ ∼ 9.9 x 10⁶ or less, a water-soluble high molecular compound comprising a acrylamide as a constituent monomer is not contained therein, and lithium chloride is contained as an essential component;
(2) the essential components comprises at least one selected from a group consisting of (A) water-soluble and/ or water-swelling natural and/ or synthetic high moleculas compound excluding a water-soluble high molecule compound having a molecular weight less than 3.0 x 10⁶ ∼ 9.9 x 10⁶ when measured by the intrinsic viscosity method and comprising acrylamide as a constituent monomer; (B) a mixture of any of zinc carbonate, zinc hydroxide, zinc gulucuronate, calcium lactate, calcium glycerolynate, calcium gluconate, ferrous nitrate, ferrous lactate, ferrous gluconate, copper gluconate, calcium chloride,a mixture of water insoluble calcium salt and a glucono-δ-lactone, and further at least a salt of 2-amino-2- methyl-1-propanol and/ or alkali metal of acids such as hydroxy acid of phosphorus , citric acid, lactic acid, DL-malic acid, fumaric acid, succinic acid, gluconic acid; (C) carbonate and/ or bicarbonate of alkali metal; (D) acids such as citric acid, lactic acid. DL-malic acid, fumaric acid, succinic acid, gluconicacid, and hydroxy acid of phosphorus; (E) activated carbon, and (F) anticeptics, germicide, antibiotics, mildew proofingagent, tickcide, pesticide, agricultural chemicals, flame retardant, and sodium sulfate anhydride,
(3) a amphoteric the high molecule compound described in (A) above having a molecular weight of 1.0 x 10⁷ or more, comprising a (meta)acrylamide as a constituent monomer, and also having a molar ratio between anion groups and cation groups in a range from 30/70 to 70 /30,
   is contained therein;
(4) the essential component contains glyoxal and/ or N-methylolpoly-acrylamide less than 3.0 x 10⁶ or prefably less than 1.0 x 10⁵;
(5) the high molecule compound in (A) is at least one of an extract from a high molecule compound of sea weeds such as gum agar, alginic acid, carrageenan, glciopeltis glue, or the like, guar gum, gum Arabic, gum tragacanth, gum ghatti, chalaya gum, lowcust bean gum, tamarind gum, xanthan gum, Kon'nyaku powder, casein, gelatin, pectin, hyaluronic acid, collagen, crosslinked, ethylenically unsaturated polymer and salt or derivative thereof;
(6) any of anticeptics, germicide, antibiotics, mildew proofing agent, tickcide, pesticide, agricultural chemicals, and flame reterdant is at least one selected from a group consisting of quarternary ammonium salt of fatty acid, benzarconium chloride, benzetonium chloride,betaine based amphoteric active agent, halogenated organophosphorus compound, organophosphorus compound, piresroide-based compound, carbamate-based compound, and
(7) the chemical is prepared into at least one of (a) a form prepared by casting a solution obtained bydissolving and/ or swelling the chemical in water and gelled; (b) a form prepared by putting a solid in a gas-permeable bag or a vessel; (c) a form prepared by pouring the solution into a vessel and/ or a vessel containing textile and/ or other water-absorbing material therein; (d) a form prepared by impregnating and/ or spraying and/ or coating the solution in, over, or on textile, other organic material, inorganic material, or other appropriate material and then drying it; and (e) a form to be used by sprinkling and / or spraying and/ or coating in a building where any of anticeptics, germicide, antibiotics, mildew proofing agent, tickcide,pesticide, agricultural chemicals, flame reterdant, and other useful but toxic agent is used, or for plants, soil, and cattle for which the above chemicals are used; and further the present invention relates to an air refreshing method using at least one of (a) a method in which a surface of the air refreshing agent is placed in a still space, (g) a method in which a surface of the air refreshing agent is placed in a space with an air flow generated by a fan, (h) a method in which air is contacted to or passed through a layer in which the air refreshing agent had been placed; (i) a method in which a solution containing the air refreshing agent therein is sprayed in and contacted to a space and/ or over an endlessly rotating porous sheet; (j) a method in which a solution containing the air refreshing agent therein is impregnated in an endlessly rotating porous sheet and then the sheet is pressed to contact the air refreshing agent to air; and (k) a method in which the air refreshing method is impregnated and/ or coated on a porous substrate.

### DETAILED DESCRIPTION OF THE PREFERRED EMBODIMENTS

Configuration of the present invention is as described above, and essential components used to solve the problems described above, composition, usage, and effects of chemicals for the same are as described below.
(a) Solubility of lithium chloride in water at 20°C is around 44 %, but the chemicals used by the present invention can be used in various forms such as a liquid phase, a sprayed mist state, a gel, powder, granulated powder, molds, a coated film, a state in which the chemicals are sprayed over or dried on a substrate such as textile. An object of use of the air refreshing agent as described above is to prevent effects of the chemicals from being lost or becoming lower because of early drying, solidification, diffusion thereof to a space due to drying of the thereof caused under a low temperature of 0°C or below, a normal temperature, and a high temperature as experienced, for instance, in a fire and an air flow generated by a fan, and to remarkably improve a use factor of the chemicals. The effect for preventing drying is far more excellent as compared to that provided by lithium bromide, calcium chloride, glycerol, or water-absorbing high molecule compounds. The toxicityappears when a dose of 1.06 g is injected into an LD50 mouse, so that the toxicity is far lower than that of lithium bromide and the agent little contaminates environment. This agent is very salty, so that it can be expected for a person having low discernment to be prevented from eating the agent by mistake, and moreover the boiling point of lithium chlcride is 1360°C, so that diffusion of the agent under normal conditions for use never occurs during storage or use thereof, and further decomposition in an alkaline range and ion exchanging never occur unlike calcium chloride which has highly absorbent to moisture. An aqueous solution of the high molecule compound according to the present invention or gel obtained by dissolving the compound in water or a gel may sometimes show high adhesiveness to a particular air contaminant, and the adhesiveness further increase when it contained lithium chloride a little or by up to around 15 %. The reason is that , even if lithium chloride is added to an aqueous solution of a high molecule compound, generally solidification does not occur, and for this reason the compound can be mixed in a wide range, and that, because of this moisture-absorbing effect, an interface between the agent and the air is always kept in a moistened state, drying and solidification of the chemicals is retarded or prevented, and effects of the chemicals are maintained for a long period of time. For instance, if dense smoke of cigarett is intermittently contacted to a cast and gelled made of 2 % agar and 10% lithium chloride at 20°C in an air flow generated by a fan and having a flowing velocity of 2 m/sec, a large quantity of tar is absorbed onto a surface of the gel, and in several days the tar diffuses to the entire gel, which can visually be checked because the color changes to brown. This capability of absorbing tar continues for more than 6 months with a reduction ratio of weight kept in a range of 50 + 10 %, but if this experiment is executed only with a gel of agar, the gel is dried and solidified within around 7 days, and the capability is lost. If any of the various compounds as described or germicide or the like is added to the gel, the function to capture and retain a large quantity of hazarduous aerosol such as dust, pollen, suspended spores, various types of mal odorous substances, and poisonous gasses is given. Even if the same quantity of glycerol is used, the specific effect for prevention of drying can not be obtained.
(b) Even if, for instance, sodium bicarbonate or zinc carbonate is put into a non-woven fabric bag and it is placed in a space where a poisonous gas having acidity such as SOx, NOx, hydrogen sulfide, or propionic acid is present, or even if such a material as succinic acid or ferrous sulfate is similarly placed in a space where a poisonous gas having basicity such as hydrazine, ammonia, or organic amines is present or in the air flow as described in (a)above, the reaction velocity between the gases and the solid chemicals is very slow excluding a case where the humidity is very high, but if lithium chloride is mixed in the chemicals by 3 % or less, a neutralizing or a double complex reaction is promoted, and the performance as an air refreshing agent is remarkably improved. Herein if any of the carbonates having basicity is mixed with a chemical having acidity such as an organic acid or a ferrous sulfide, further lithium chloride is added to the mixture , and the resultant mixture is placed in the same manner as described above in an air flow generated by a fan, a removal ratio for all the air-contaminating gases having both the acidity and basicity is remarkably improved as compared to a case where lithium chloride is not mixed. Also if 10 % succinic acid, 10 % zinc hydroxide, and 2 % lithium chloride are mixed in a 5 %suspension of activated carbon from coconut palm shell with pH of 10.5, the air refreshing capacity for a wide ranged air-contaminants containing gasses having acidity, basicity, or non-ionicchange is remarkably improved.
(c) As analogous compounds which is amphoteric and high molecule compounds containing (meta)acrylamide as one of the essential monomer of the present invention and has a molecular weight of1.0 x 10⁷ or more and in which a molar ratio of anion groups against cation groups is in a range from 30/70 to 70/30, there can be enumerated many types of combination of anionic and cationic monomers respectively, but it has empirically been impossible to prepare such a high degree of polymerization by the copolymerizing method. To briefly describe the synthesizing method employed in the present invention, for the purpose to obtain a molecular weight of 1.0 x 10⁷ or more and to obtain the remarkable air refreshing capability, the starting material should be an anionic copolymer having a molecular weight of 1.7 x 10⁷ or more, and preferably of 2.0 x 10⁷ or more. The molecular weight was measured by measuring the intrinsic viscosity according to the Mark-Houwink-Sakurada method, because it was considered that the method was suited for measurement of a molecular weight of the macro-molecules as described above. At first an aqueous solution of this substance with the concentration of 5 % or less, preferably of 4 % or less,and most preferably of around 2 % is accurately prepared, slowly agitated according to the known method so that any inter- and intra-molecular cross-linkage will not occur therein with formalin by the molar number in a range described above added to the anionic group to have it methylolated and then dimethylamine added to the methylolated anionic poly(meta) acrylamide to have it dimethylaminomethylated, thus a amphoterically charged terpolymer with high stability being obtained. The product with concentration of 2 % has high viscosity of, for instance, around 50,000 mN·sec (cps), and this aqueous solution is highly hard to dilute with water due to the effects by hydrogen bonds and electrostatically attracting force effecting in the molecular, but if water is added little by little and the mixture is agitated slowly, the solution can be diluted without the drop in molecular weight Herein, if the molecular weight is 2.0 x 10⁷ or more and a sum of the cationic group and the anionic group exceeds 40 molar %, the viscosity becomes too high because the inter- and intra-molecular electrostaticilly attracting force becomes too strong, but the electrostatically attracting force becomes lower if lithium chloride coexists there, and then it becomes easy to manufacture, handle, and use the substance. A molar ratio between the anionic group and cationic group is set in a range from 30/70 to 70/30 according to a purpose of use thereof, and the aqueous solution with the molar ratio in a range from 40/60 to 60/40 forms around 20 % combined water when the aqueous solution is dried under a temperature of 110°C for 5 hours to form a film thereon, and the film dried under a room temperature is water-swelling but insoluble due to formation of an inter- and intra--molecular salt-formative reaction. However, if the molecular weight is less than 8 x 10⁶, the substance does not become insoluble to water like other charged high molecule, and is dissolved in water. As the molecular weight increases, the water-swelling characteristics is maintained, and the capability for absorbing air contaminants at a low concentration becomes substantially higher. It was found through observation of a photograph taken by a scanning electronic microscope and magnified by 5000 time or more that the film prepared from an aqueous solution of this amphoteric high molecule compound and dried under a room temperature or fine grains of the sprayed mist under a room temperature is porous. Main embodiments in a case where a small quantity of any aqueous soluble organic solvent with a boiling point lower than that of water such as ethyl alcohol is added thereto and the mixture is used as an air refreshing agent are as described below. {a} Prevention of environmental contamination by useful hazardous materials such as the agricultural chemicals as described above: For instance, when a 5 to around 200 ppm aqueous solution of this high molecular compound containing lithium chloride by around 1 % therein is sprayed over textile, woody materials, tatami (Japanese matting made from special grass), wallpaper containing hydrophilic filler, interior covering for building, zeolite, soil, plants, or other materials and is then dried to capsulize the useful hazardous substance within this high molecular compound because of its micro-capsulating effect so that the useful hazardous material will gradually be released, it is possible to prevent the useful hazardous substance from unnecessarily and rapidly leaking into a building or into the environment, to maintain its efficacy for a long period of time, to improve the capability to adhere to any dry object for processing, to give a directionality to the substance in spraying for the purpose to prevent diffusion of the substance by adjusting the high molecule compound to make a particle diameter of the mist larger, and to reduce leaching by rainwater or bedewing, thus a use rate of the useful hazardous substance being reduced, environmental contamination being prevented, and also physiological gas influence to workers, residents, and cattle being reduced. {b} For instance, if textile is treated with an aqueous solution containing 10 ppm lithium chloride, 5 ppm amphoteric high molecular compound having a molecular weight of 2.0 x 10⁷, and 700 ppm benzarconium and then the textile is dried and used for covering a patient lying on a bed when the patient evacuates the bowels, it is possible to prevent the mal odor or disease germs from spreading in the sickroom. Also it is possible to refresh air inhaled by a patient if thepatient wears a mask made from Rayon or cotton treated asdescribed above
{c} Purification of air by using non-woven Rayonfabric with the chemical solution described in 2 above impregnated therein and then dried as a filter for anair conditioning machine.
{d} Refreshing of an exhaust gas realized by, for instance, refreshing an exhaust gas from a compost plantcontaining mal odor, poisonous gasses, germs of harmful microorganisms, and fine particles each at a high concentration bycontinuous or intermittent spraying an aqueous solution of the high molecule compound asdescribed in 2 above, yet not containing germicide therein so that the diameter of the mist will be in a range from 10 to 30µm onto a surface of an endless belt made of textile and having high strength, said endless belt incorporated in a fan, to let an air flow contact the belt, and then by discharging the liquified solution into a sewage system purificatory cistern or burning the discharged liquid.
{e} Refreshing of air in a space in which air contaminants are present such as a lavatory, a sickroom, or a smoking room applied by injecting an aqueous solution containing the amphoteric high molecule compoundaccording to the present invention and having a molecular weight of 2.3 x 10⁷ by 3 ppm and lithium chloride by 1 ppm from a pressure-resistent aerosol spray made of aluminium with LPG or dimethyl ether sealed therein as a propellant or by injecting the aqueous solution without any propelling agent from a plastic spray pump.
{f} Refreshing of air in a burning room during a fire by injecting in a mist state an aqueoussolution containing the amphoteric high molecular compound having a molecular weight of 2.3 x 10⁷ by 3 pp, lithium chloride by 10 ppm, and a fire-proof agent such as ammonium bromide/ or ammonium phosphate by totally 50 ppm in a burning room in an early stage of a fire tosupress the smoke and retard flashover
{g} Refreshing of air in a room to reduce a comentration of hazardous volatile organic compound easily emitted in a newly built house or building; For instance, by spraying a sufficient quantity of an aqueous solution containing the amphoteric high molecule compound according to the present invention by 10 ppm and lithium chloride by 2 ppm over carpet, "tatami", or a sofa and then drying the solution, or by coating the aqueous solution described above over the entire surface of plywood plate in which urea resin is used as an adhesive, it is possible to remarkably improve the reaction ratio with low concentration formaldehyde emitted thereof because the film contains moisture and this moisture works as a plasticizer for the high molecule compound film, so that generation of cracks in the film or separation thereofdue to elongation or shrinkage of the film caused by the change of humidity can be prevented. Further the above purpose can be achieved putting the activated carbon in (i) above and the cast and gelled type of chemical describe in (e) above in a small bag made of unwoven cloth and placing the bag in a confined residence or in a fan with a battery-driven propeller fan attached thereto and continuously driving the fan.
{h} Refereshing of air realized by pouring a useful hazardous substance such as germcide and the amphoteric high molecule compound having a density of 0.2 % and a molecular weight of 2.4 x 10⁷ into a plastic vessel with sponge placed therein, placing the vessel in a sickroom or a compost plant, and continuously sending wind to the vessel.
(d) An aqueous solution of the high molecule compound according to the present invention and containing lithium chloride therein, particularly the aqueous solution with a positive activator such as aliphatic tetra-ammonium salt, benzarconium chloride, or benzetonium chloride each amphoterically chargeable, having a molecular weight of 1. 0×10⁷ or more, at a low concentration for instance in a range from 10 to 50 ppm as a solid base, and with a pH value of around 10.5, or at a low concentration of around 0.2 % as a solid base, or a betaine type of amphoteric surfactant dissolved therein shows a strong sterilizing effect or a germ-suppressing effect to harmful microorganisms such as many pathogenic bacteria, bacteria causing putrefaction, or molds. The biological mechanism has not been clarified yet, and it can be presumed that the capability of lithium chloride to capture and maintain moisture improves a bacteria capture ratio. With the treatment as described above, it is possible to obtain an additive to a liquid used when a cast and gelled product or a solution is poured into a vessel together with textile or other water-absorbing material, and also to obtain filter excellent in sterilizing capability, deodorizing capability, and capability for capturing fine dust by impregnating the aqueous solution in water-absorbing woven cloth or non-woven cloth and then during the cloth. As a useful hazardous substance such as a tickcide preparation, a pesticide preparation, or agriculture chemicals, almost all of commercially available products can be used for the purpose according to the present invention, The representative examples include, but not limited to organophosphorus-based chlorpyriphos (CAS No. 2921-88-21), ECP (CAS No. 97-17 6), EPN (CAS No. 2104 64- 5),ESP ( CAS No. 2674 - 91 - 1), MEP (CAS No. 12214 - 5), carbamate-based MIPC (CAS No. 2631 - 40 4), MPMC (CAS No. 2425 - 10 - 7), MTMC(CAS No. 1129 - 41 - 5), NAC (CAS No. 63 - 25 - 2), silver powder or any compound thereof, and a mixture thereof.
(e) Glyoxel and N-methylolated acrylamide molecule weight less than 3 x 10⁶ , prefably less than 1 x 10⁵ , which emit formaldehyde has a high deodorizing effect, but has also toxicity, so it is difficult to use these as a raw material for household products. However, of the high molecule compounds described above, a polyacrylamide-based high molecule compound having a molecular weight of 1.7 x 10⁷ or more is used together, the poisonous substances are efficiently enclosed within the high molecule due to moisture maintained therein, so that the two substances can be used safely.
(f) Further even if glyoxal or N-methylol polyacrylamide is not used, by using the water-soluble calcium saltsenlisted in (c) of Claim 1, as calcium hydroxide is not separated under pH of 9 or less, so that it is effective for deodorizing hydrogen sulfide, melcaptans, or lower aliphatic acid.
(g) Also in a case where at least one of zinccarbonate, zinc hydroxide, calcium salt, ferrous salt, hydroxy acids of phosphorus, acids, alkali metal carbonate or alkali metal bicarbonate, and activated carbon is included and pH is adjusted to an appropriate range, it is possible to give the capability for specifically capturing a specific poisonous gas by the strong reactivity and buffering capability of these chemicals.
(h) Many types of hazardous aerosols such as soot, smoke of cigarette, fine perticulate of asbestos, dust, pollen, mine powder, or oil droplets in a kitchen are easily diffused by a repelling force generated from positive or negative charge specific to each substance in a space and hardly precipitate by gravity, if these materials contact the chemical of the present invention and is electrostatically neutralized with its diffusing force lost, and also when a low concentration aqueous solution of the various types of polymers as described above or a gel thereof is used, the concentration rapidly increases because of the adhesion effect with the precipatiting velocity acceleratedly increased, so that the materials are easily captured. By using a surfactant, the effects as described above can further be improved.
(i) Different from the conventional technology, the chemical according to the present invention contains moisture which is hardly evaporated, so that the surface is liquidous, bedewed, or fully moistened, and for this reason not only mal odorous substance in the molecular state and hazardous gasses, but also harmful aerosols such as dust and microorganisms are well absorbed, which enhances the effect of the chemical formulated therein.
(j) In a case where the germicide as described above is not used, anticeptics and mildew proofing agent are required, but for the purpose as intended in in the present invention,it is desirable to use any of 10 to 70 % ethyl alcohol, alkali salt of sorbic acid, paraben, alkali salt of benzoic acid, alkyl ester of p-oxybenzoic acid (Number of carbon atoms in the alkyl group is 2 to 4), anddehydroacetate or a salt thereof.
(k) Although many types of tickcide, pesticide, and agriculture chemicals currently available in the market are used after the toxicity is carefully checked and used in such forms as solution, emulsion, or powder, the following advantages can be obtained when any of the chemicals is mixed in the formulation according to the present invention and the mixture is used in a liquid state.
(l) When injecting agriculture chemicals to plants or pesticide for termite over soil or partition walls or stractural components of woody buildings, if any of the chemicals is mixed with the chemical according to the present invention and a concentration of the high molecular compound is adjusted to a level a little higher than the limit , when the mixture is sprayed with a spray gun, mist having a large particle diameter of around 100 µm is generated, and under such conditions the agricultural chemicals or other are not dispersed into the air with the increased ratio of deposition to the object material, so that it is possible to prevent or reduce environmental contamination and inhalation of the chemicals by workers, and also it is possible to reduce a quantity of hazardous gasses resulted after use thereof. Also by sellecting a high molecular compound, the level of the water resistance after drying, so that also a period of efficacy after its use can be designed beforehand. Also it is possible to decide the water content to make it easier to deposit on woody materials or trees for capsulized agriculture chemicals or other substances within the high molecular compound according to the present invention to deposite on woody materials or trees.

### DETAILED DESCRIPTION OF THE EMBODIMENTS

Next description is made for embodiments and examples for reference of the present invention.

### EXAMPLE1 AND REFERANCE 1-2

A 15cm x 15cm sized board of 3-ply lauan based plywood of 2.5 mm thick, bonded with an urea resin (Reference 1), the same board both-side coated at the coverage rate of 60 g/m for each side by a spongy roller with an aqueoussolution containing 50ppm as solid of an amphoteric copolymer (which is described as Copolymer A hereinafter) prepared through a Mannich reaction (i.e. the same molar ratio of dimethyiaminomethylation as the following acrylic acid) to an anionic copolymer of molecular weight of 1.7x 10⁷ and the consistuent monomers, acrylamide/acrylic acid, being 85/15 on molar basis, and 2g per litre of potassium sorbate (Reference 2),and another same board coated in the same way with an solution containing 2g/litre of lithium chloride (Example 1)were dryed at 60°C for 15 minutes in an oven and cooled to room temperature respectively. Each board was then put separately into a nylon coated polyethylene bag which has a value. Each bag was heat-sealed, and then five litres of fresh air of 87% R.H. at 20°C was pumped into each bag. After each bag was allowed to stand at 35°C for 8 hours, the formaldehyde cotent in each bag was as follows when measured by the gas-detector tube method.
Reference 1 : 12ppm (by volume)
Reference 2 : 1.6ppm
Example 1 : Not detected (which is described as N.D. hereinafter)

### EXAMPLES 2 to 4 and REFERANCE 3 to 4

Each component of the formulation as shown in Table 1(Unit: g) was put in a glass beaker, then ion-exchanged water was added thereto to the total weight of 1 kg, the mixture was heated upto 80°C for dissolving, and after confirming that each component had become clear, each 100g solution was casted into 10 plastic cups respectively each having size of 43 mm high ,71 mm upper diameter, 64 mm bottom diameter, and then gelled after allowing to cooled at 30±3°C for 12 hours in a room not having been sterilized without the cover on each vessel after it was confirmed that allpreparation had been solidified into light yellowish transparent or semi-transparent gel, , the cups were kept for 30 days, and then the tests were condnctedunder the conditions shown in Table 1.

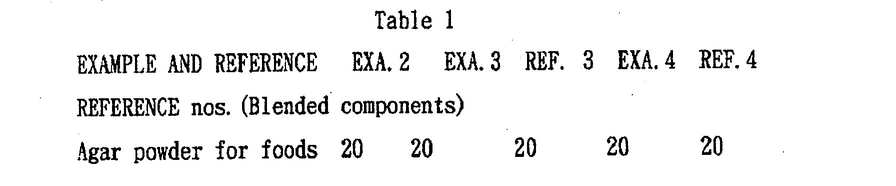

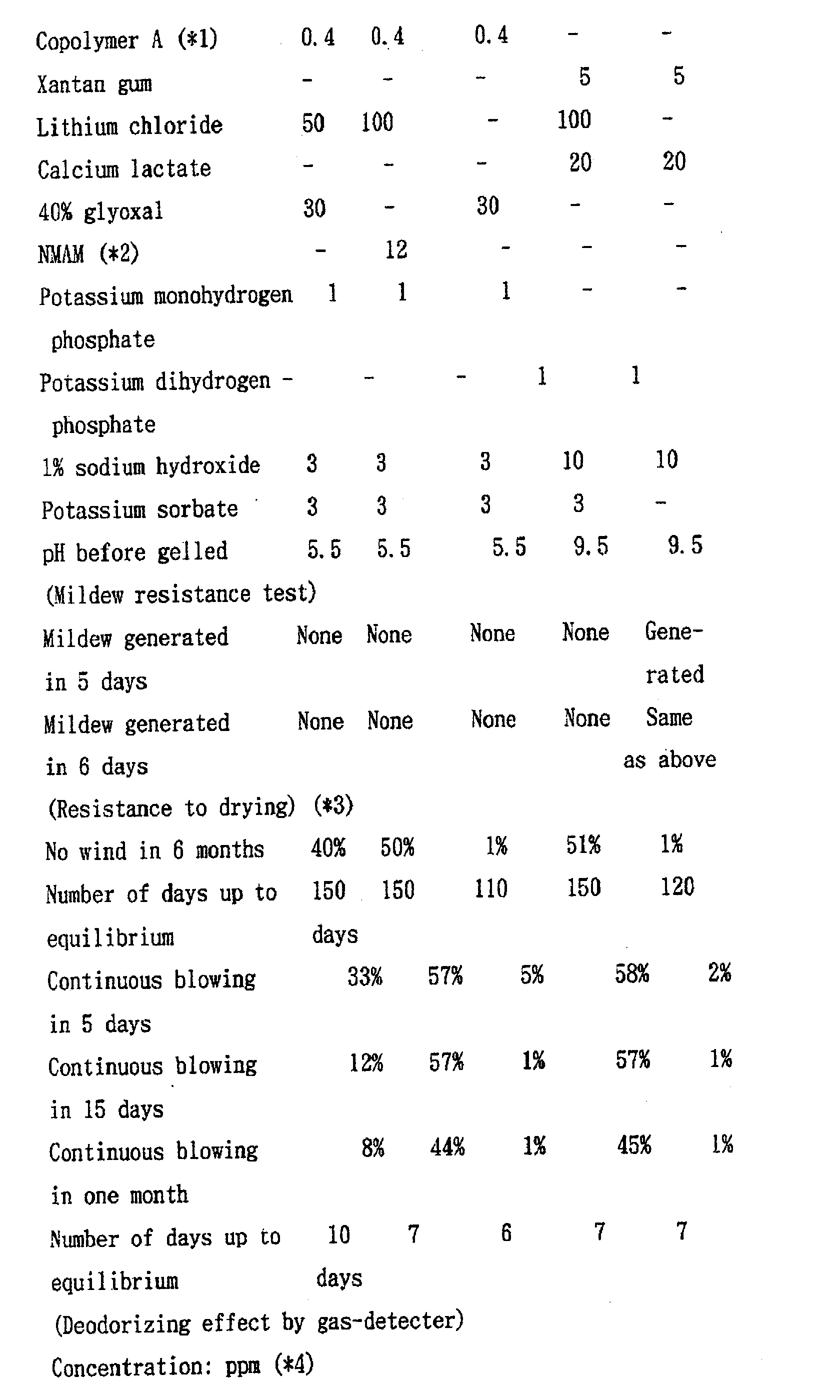

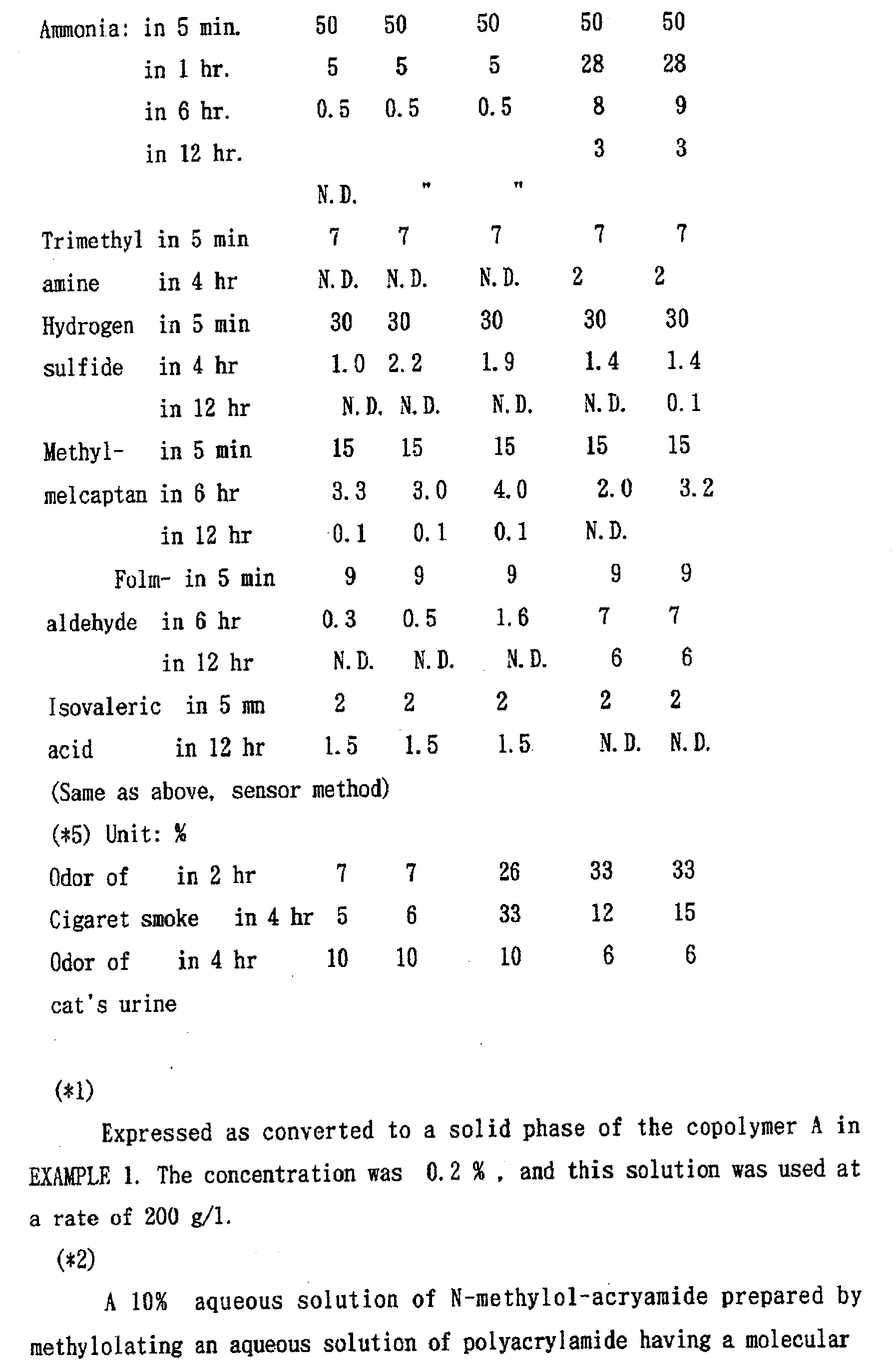

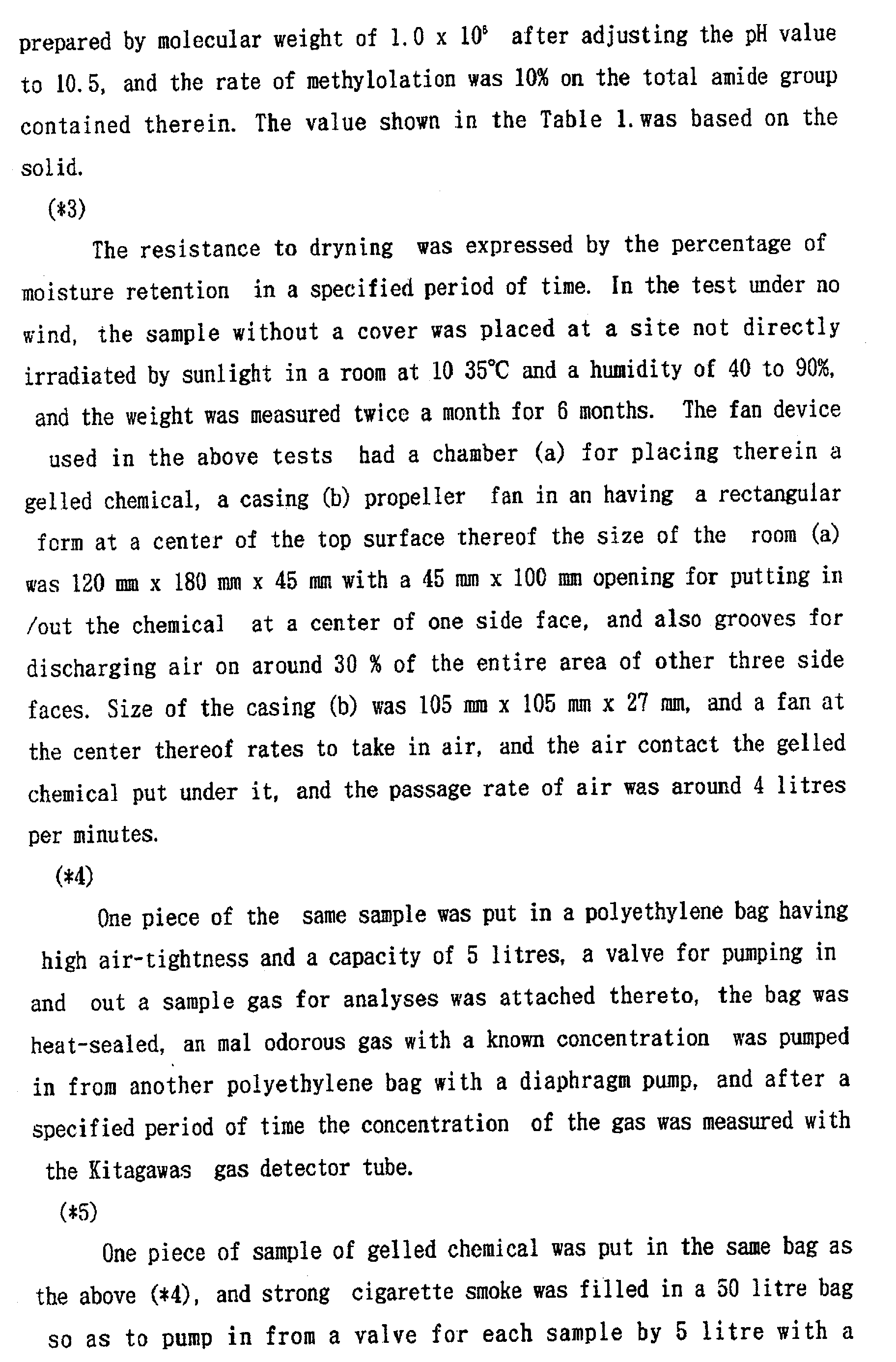

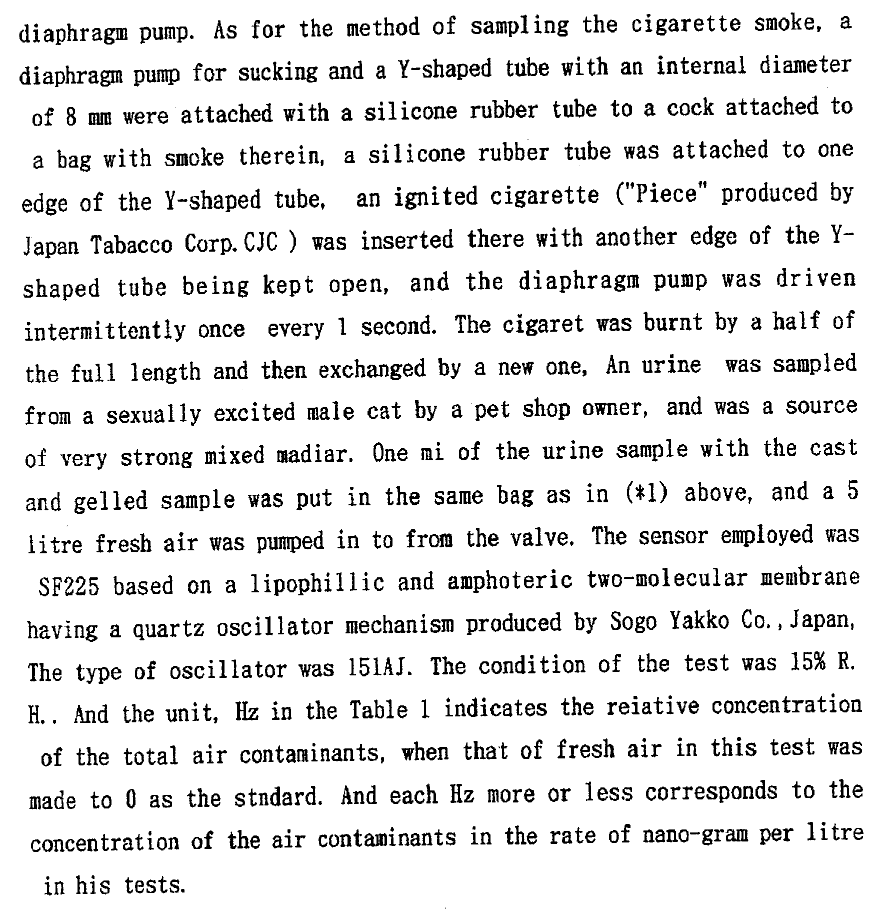

### EXAMPLE 5 AND 6

An aqueous solution containing 3ppm of sodium hyaluronate of which molecular weight was 3 million, 5 ppm of Copolymer A in Example 1 above, 1% of lithium chloride and 0.15% of benzarconium chloride (Example 5), and another solution containing the same components but the concentratin of benzarconium chloride was 0.025% (Example 6) were adjusted to ph 11.0, by adding a slight quantity of 2-amino-2-methyl-1-proponol (described as AMP hereinafter) respectively, and then determination of the capability for sterilizing the eight germs described below and one germ causing putrefaction were conducted at Japan Food and Drug Safety Centre (a foundational juridical organization ). In these testings, 5 ml of each liquid was put in a small test tube, 0.1 ml of a germ containing liquid was treated with each testing liquid, 1 needle hook of the sample was taken out after 15 sec, 30 sec, 1 min, 2 min, 5 min, 10 min, 30 min, 1 hr, and 2 hr respectively, and then the bacteria was incubated on the BHI liquid medium, while yeast and mold were incubated on a GP medium, and further cultivated for 48 hours at 35°C and then kept for 1 week under 27°C.The determination was done by checking whether any growth wasobserved or not by a microscope.

| Name of the tested microorganism and growth | EXAMPLE 5 (Germ) | EXAMPLE 6 (Germ) |
|---|---|---|
| Staphylococcus aureus | None | None |
| Staphylococcus aureus (MRSA) | None | None |
| Escherichia | None | None |
| Pseudomonas aecolicruginosa | None after | None after |
| | 30 second | 15 minutes |
| | | |
| Streptococcus faecalis | None | None |
| Bacillus subtilis | None | None |
| (Petrefative germ) | | |
| (Yeast) | | |
| Candida albicans | None | None |
| (Molds) | | |
| Aspergillus fumigatus | None | None |
| Trichlophyton mentagrophytes | None | None |

From a result of these tests , and taking into the data disclosed literatures , it can be considered that the capability of sterilizing effects for the bacteria are more excellent than those of benzarconium chloride alone at a low concentration, and these result showed that the chemical can be used in 4 types of usages according to the present invention, and especially the results of tests conducted in a cleaning plant showed that the preparation according to EXAMPLE 5 was extremely effective as a continuously sterilizing agent used for diapers, sheets, and cloths used by patients in hospitals, which may cause intra-hospital infection, when the materials are washed, dried, and maintained until the next use, or as a deodorizer against water-insoluble mal odorous materials substantially remaining in wasted water after treatment of the materials described above, and that consequently the preparation was extremely effective for preventing the contamination of air by spraying or damages to skin of workers or users due to the excessive use of germicides.

### EXAMPLE 7

A solution containing 10ppm of sodium hyaluronate having a molecular weight of around 3.0 x 10⁶ , 1 g of lithium chloride, 1 g of benzarconium chloride, 1 g of potassium dihydrogen phosphate, and sodium hydroxide in its total weight of 1 Kg with a pH of 8.2 (under 20°C) was impregnated in a stich-bonded type non-woven fabric based filter mainly made of rayon with the wet pickup to around 300 % by padder(mangls), and then it was dried for 7 minutes at 120°C. The unwoven cloth thus treated is suited to be used as a material for a layer of mask contacting a human face for deodorizing mixed mal odors and for capturing a hazardous aerosol or dust. pH of the above solution was 7.0 + 1.5

### EXAMPLE 8 and Reference 5

Using an aqueous solution prepared by adjusting pH to 10.5 with AMP at 20°C of a solution containing 300ppm of the copolymer A in EXAMPLE 1 above, 10g of lithium chloride , 1g of potassium sorbate, and 1g of potassium dihydrogen phosphate in the total weight of 1 kg , the processing under the same conditions as those in EXAMPLE 7 was applied to the same non-woven fabric. (EXAMPLE 8 )

In order to compare the capability for collecting suspended particulales of non-woven fabric without treatment, (Reference 5), the two types of non-woven fabric, one treated and not treated as described above, were adhered to a top surface of a funnel, and the funnel was used for sucking external air at a site with a heavy traffic volume with a diaphragm pump having the sucking capability of 15 l/min set thereunder. A result of visual check of surfaces of the two types of non -woven fabric after the test showed the difference between these fabric was clear in terms of collecting contaminants as soot which can be regarded as that contained in an exhaust gas from diesel engines or other types of fine particles. It was determined that the treated fabric has the capability for collecting the air contaminants equivalent to that of a high voltage electrostatic device currently popularized. It was also recognized that, when the non-woven fabric according to EXAMPLE 8 was attached to an air exhaust pipe (with a displacement of 2.5 m3) of a garbage composter for business use while driven, the malordor concentration was 200,000 as defined in the malodor measuring method by the Japanese Envirenment Protection Agency reduced to around only 15, 000.

### EXAMPLE 9

A stich-bonded non-woven fabric mainly consists of rayon having a weight of 120g/m² was padded by a mangle roll with a liquid containing ten times by weight of each component than that of the formulation of the Reference 5, and the wet pickup was around 300 % , and then dried for 7 minutes at 120°C was well suited for uses as a material of sheets for disposable cloth or for sterilization as well as of a filter for air-conditioning in a working site where infection by microorganisms may occur.

### EXAMPLE 10

A high viscosity aqueous solution containing 2g of xantan gum (Echo gum T produced by Dai Nippon Seiyaku Co.) 110g of lithium chloride , 3g of benzetonium chloride, and 2g of potassium sorbate having with the pH adjusted to 11.0 by adding AMP (Solution A) was put into plastic vessels according to EXAMPLE 2 to 4 after a round layered material made from non-woven fabric used in EXAMPLE 8 was put in for prevention of leakage, and the vessels were put in a fan having around 2 times larger size as compared to that used in EXAMPLE 3 and 4 together with the cast and gelled deodorizer used in EXAMPLE 3, and this set was placed in a nursing room of a hospital for aged people, where a ventilation test was performed continuously for 50 days, but in any case, because of the feature of this preparation according to the present invention that evaporation of moisture was suppressed largely, it was observed that 44 % and 41 % of the test liquids remained, and also it was recognized by many nurses that mal odor as felt through the olfactory smell in the hospital remarkably reduced. It was observed that lithium chloride was segregated from the solution (A) and the color changed to dark , but presence or growth of any microorganism was not recognized in a result of observation of the solution with a microscope as well as in a result of incubation of this solution according to the methods employed in EXAMPLE 5 and 6. From this fact, it was confirmed that microorganisms suspended in the air contacted to the solution A during this test period had been completely sterilized.

### EXAMPLE 11&12 and REFERENCE 6

In a cylindrical steal can of 12 cm diameter 30 ml of toluene was put in and ignited . The site was enclosed by 4 sheets of vertically erected glass plate each having a size of 40 cm x 50 cm. When a large quantity of sout was generated, water was sprayed in the horizontal direction from a point 40 cm away from an upper edge of the glass plate so as to the particle diameters distribution becomes in a range from 10 to 50 µm. And the condition was photographed how the smokes was suppressed, but any remarkable effect for suppressing smoke was not observed. ( Reference 6) The same test was conduched by using 2ppm solution of a copolymer B which has a molecular weight of 2.4 x 10⁷ and similar to the copolymer A prepared by the method as described in EXAMPLE 1 but was different in that the carboxyl and the dimethylaminomethyl groups were 20 mole percent respectively 1 ppm of lithium chloride, and 0.1% potassium monohydrogen phosphate. In the experiment the combustion was maintained, but a clear effect for suppressinging smoke was photogrpahed. (EXAMPLE 11) When additionally 2 % of ammonium bromide was added to the liquid used in EXAMPLE 11, conducting the same experiment , it was recognized that not only the smoke was suppressed but also the combustion of toluene was extinguished. This result indicates the possibility of use of this chemical as a smoke and fire fighting agent.

### (EXAMPLE 12)

### EXPERIMENT 13 and Reference 7&8

Two pieces of each cigarette ignited were placed in array on an ash tray in a dark room with no wind so that two flows of smoke would go up in parallel at least around 80 cm away from each other, and 2.1 g of water was sprayed for 2 seconds from a spray nozzle for a particle diameter distribution from 2 to 30 µm from a point around 50 cm above the point where the two smoke flows crosses each other, and also 40 cm away in the horizontal direction with the situation recorded by a video camera, but only slight disturbance was observed in around 40 seconds after start of spraying. (REFERENCE 7) In a case where the same experiment was done using an lppm aqueous solution containing the copolymer according to EXAMPLE 11, 1 ppm lithium chloride, sprayed mist started extinguishing the two flows of smoke in about 20 seconds after start of spraying, the effect was maintained for around 30 seconds, and during this period of time the spray mist moved in various directions to show a typical Tyndal phenomenon, and the fact that the mists promoted precipitation of smoke was recorded by the video camera, and also the deodorizing effect was confirmed olfactory. (EXAMPLE 13) In the experiment executed by using the copolymer C which was the same as copolymer A excluding the point that the molecular weight was 8 x 10⁶, the fact that Tyndall phenomenon was started in 35 seconds after start of spraying was recorded, and although the effect was maintained for only around 20 seconds, the smoke suppressing and deodorizing effect was substantially lower during this time. (REFERENCE 8)

The air refreshing agent according to the present invention is manufactured by basically blending the base materials each having extremely low toxicity, and seldom causes a chemical reaction generating any hazardous substance, so that almost all examples of the present invention are every safe. Also in a case where glyoxal or formaldehyde having the possibility to emit harmful gas or gasses in a step of solidification like the cast and gelled air refreshing agent or NMAM having the possibility to emit other types of volatile raw materials is used together. with the method according to the present invention, moisture is fully maintained and enclosed within a vessel.

In a case where this chemical is injected into a space so that the particle diameter will be in a range from 1 to 100 µm, it is possible to effectively remove various types of air contaminants including suspended particle each having a particle diameter of 100 µm or less through the mechanisms of acceleration of precipitation velocity due to the increase of the particle diameter with the injected mist deposited thereon, removal of electric charge which is a cause for repelling property of aerosol, or chemical or physical absorption. Also it is possible not only to efficiently achieve precipitation and capsulization of hazardous aerosol such as smoke of cigarette or sulfuric acid mist, fine particle of asbestos, suspended particles in an exhaust gas from a diesel engine, pathogenic germs or spores thereof, all of which has been proved as harmful from a viewpoint of public health, but also to efficiently achieve sterilization, supression, prevention of proliferation which may cause opportunistic infection. Also in a case where a useful poison such as a low molecular weight agriculture chemical and the mixture are sprayed over or coated on an object for treatment, the substance such as the agriculture chemical is micro-capsulized with the capability for gradual release added thereto and the period of efficacy prolonged, and further the ratio of deposition onto an object for processing in use is improved, and also rapid diffusion into a space and leaching out the component due to rainfall or bedewing, and inhalation by workers can be prevented or reduced, which contributes to refreshimg of air. Any component other than water does not evaporate from the chemical formed into a gel state in a vessel according to the present invention, and the chemical efficiently absorb harmful aerosol chemically or physically from mal odorous materials and further is excellent in the resistance against drying, freezing, and wind, which makes it possible to provide a novel air refreshing system.

The conventional type of air refreshing technology has been developed under the initiative by companies in the fields of apparatus industry and electric equipment, so that the main stay in the technology has been depending on use of ozone with high toxicity, activated carbon with organic amines which can hardly be aborted or regenerated and has a high toxicity deposited thereon, or the like, so that maintenance and management thereof are very difficult. Further in the apparatus based on the combustion method or scrubber method, the size is very large with large energy consumption. Feature of the present invention is not suited to large scale processing of air contaminants such as those in iron mills or plants for petrochemical industries, but can be applied to many small scale sites having the possibility that various types of air contaminants may be discharged without installing a large scale apparatus or system.

Further different from the masking method using aromatic chemicals which is at present the main technology in this field, relative advantages provided by the air refreshing method according to the present invention can scientifically be proved and are mote persuasive for users, and it is possible to select and provide a chemical, a method, and a device suited to each user's site.

### POSSIBILITY OF UTILIZATION FOR INDUSTRIAL PURPOSES

The air purifying agent and the air purifying method according to the present invention provides the possibility for efficiently removing contaminants including suspended particle having a diameter of 100 µm or less from mol odorous molecules having a diameter of 0.2 nm or more or poisonous gasses, and also provides an air refreshing agent with improved capability for preventing diffusion of useful hazardous substances such as agriculture chemicals into a space as well as a high adhesiveness to objects for processing because of the micro-capsulizing mechanism, and also for reducing loss due to rainfall.

## Claims

1. An air refreshing agent; wherein said agent does not contain a water-soluble high molecule compound having a molecular weight in a range from 3.0 x 10⁶ ∼ 9.9 x 10⁵ or less when measured according to the intrinsic viscosity method and containing acrylamide as a constituent monomer andfurther contains lithium chloride as an essential component.

2. The air refreshing agent according to Claim 1; wherein the essential components comprises at least one selected from a group consisting of (A) water-soluble and/ or water-swelling natural and/ or synthetic high molecule compound excluding a water-soluble high molecule compound having a molecular weight in a range from 3.0 x 10⁶ ∼ 9.9 x 10⁶ or less when measured according to the intrinsic viscosity method and comprising acrylamide as a constituent monomer; (B) a mixture of any of zinc carbonate, zinc hydroxide, zinc glucuronate, calcium lactate, calcium glycerophosphate, calcium gluconate, ferrous nitrate, ferrous lactate, ferrous gluconate, copper gluconate, calcium chloride, a mixture of water-insoluble calcium salt and a gluconodelta lacton, and further at least2-amino-2-methyl-1-propanol salt and/ or alkali metallic salt of acids such as hydroxy asids of phosphorus, citric acid, lactic acid, DL-malic acid, fumaric acid, succinic acid, gluconic acid; (C) carbonate and/ or bicarbonate of alkali metal; (D) acids such as citric acid, lactic acid. DL-malic acid, fumaric acid, succinic acid, gluconic acid, and hydroxy acid of phosphorus; (E) activated carbon,and (F) anticeptics, germicide, antibiotics, mildew proofing agent, tickcide, pesticide, agricultural chemicals, flame reterdant, and sodium sulfate anhydride.

3. The air refreshing agent according to Claim 1 or Claim 2; wherein the high molecule compound described in (A)contains at least one having a molecular weight of 1.0 x10⁷ or more, comprising a (meta)acrylamide as a constituent monomer, and also having a molar ratio between anion groups and cation groups in a range from 30/70 to 70/30, which is both positively and negatively chargeable.

4. The air refreshing agent according to any Claims 1 to 3; wherein the essential component contains glyoxal and/ or molecular weight less than 3.0 x 10⁶ of N-methylolpolyacrylamide;

5. The air refreshing agent according to any of Claims 1 to 4; wherein the high molecule compound in (A) is at least one of an extract from a high molecule compound ofsea weeds such as gum agar, alginic acid, carrageenann, gleispeltisglue, or the like, guar gum, Arabic gum, tragacanith gum, ghatti gum, chalaya gum, lowcast bean gum, tamarind gum, xantane gum, Kon'nyaku powder, casein, gelatin, pectin, hyaluronic acid, collagen, crosslinked eshylenically unsaturated polymer and salt or derivative thereof.

6. The air refreshing agent according to any of Claims 1 to 5; wherein any of anticeptics, germicide, antibiotics, mildew proofing agent, tickcide, pesticide, agricultural chemicals, and flame reterdant is at least one selected from a group consisting of quarternary ammonium salt of fatty acid, benzarconium chloride, benzetonium chloride,betaine type of amphoteric active agent, halogenated organophosphorus compound, organophosphorus compound, piresroide-based compound, carbamate-basedcompound.

7. An air refreshing agent; wherein the air refreshing agent according to Claim 1 is prepared into at least one of (a) a form prepared by casting a solution obtained by dissolving and/ or swelling the chemical in water and gelatinating the solution; (b) a form prepared by putting a solid in a gas-permeable bag or a vessel; (c) a form prepared by pouring the solution into a vessel and/ or a verssel containing textile and/ or other water-absorbing material therein; (d) a form prepared by impregnating and/ or spraying and/ or coating the solution in, over, or on textile, other organic material, inorganic material, or other appropriate material and then drying it; and (e)a form to be used by diffusing and/ or spraying and/ or coating in a building where any of anticeptics,germicide, antibiotics, mildew-proofing agent, tickcide, pesticide, agricultural chemicals, flame reterdant, and other hazardous agent is used, or for plants, soil, and cattle for which the above chemicals are used.

8. A method of refreshing air; wherein the air rub agent according to Claim 1 is used according to at least one of (a) a method in which a surface of the air refreshing agent is placed in a still space, (g) a method in which a surface of the air refreshing agent is placed in a space with an air flow generated by a fan, (h) a method in which air is contacted to or passed through a layer in which the air refreshing agent is present; (i) a method in which a solution containing the air refreshing agent therein is sprayed in and contacted to a space and/or over an endlessly rotating porous sheet; (j) a method in which a solution containing the air refreshing agent therein is impregnated in an endlessly rotating porous sheet and then the sheet is pressed to contact the air refreshing agent to air; and (k) a method in which the air refreshing method is impregnated and/ or coated on a porous base material.
